# EUROPEAN PATENT APPLICATION

(11) **EP 3 929 928 A1**
(43) Date of publication of application: **29.12.2021**
(21) Application number: 21179725.3
(22) Date of filing: 16.06.2021
(51) Int. Cl.: G16B 10/00, G16B 40/20, G16B 40/30, A01H 1/04

(54) **ASSOCIATING PEDIGREE SCORES AND SIMILARITY SCORES FOR PLANT FEATURE PREDICTION**

(30) Priority: 23.06.2020 EP 20181663
(71) Applicant: KWS SAAT SE & Co. KGaA, 37574 Einbeck (DE)
(72) Inventor: DA SILVA, Helena Sofia Pereira, 37574 Einbeck (DE); MAHONE, Gregory Stewart, 37574 Einbeck (DE)
(74) Representative: Richardt Patentanwälte PartG mbB

(57) **Abstract**

The invention relates to a computer-implemented method comprising:
- receiving (102) a set of pedigree scores (300, 512) of pairs of plant breeding units over two or more generations;
- receiving (104) an incomplete set of similarity scores (200, 510) of the pairs of the plant breeding unit pairs;
- aligning (106) the pedigree scores and the similarity scores of identical plant breeding unit pairs;
- automatically analyzing (108) the aligned pedigree scores and similarity scores for computing a predictive model (508) based on associations of the similarity scores and of the pedigree scores;
- using the predictive model for creating (112) a complete set of similarity scores (400, 518); and
- using (114) the complete set of similarity scores for computationally predicting a feature (522) of a plant breeding unit or of an offspring thereof.

## Description

### TECHNICAL FIELD

The invention relates to the technical field of genomic prediction and other forms of biological marker-based predictions. More specifically the invention describes a method that allows performing a genomic/biological marker-based prediction based on an incomplete similarity coefficient dataset.

### BACKGROUND

Genomic prediction is an approach commonly used by plant breeding companies to assess a plant's genetic merit based on scoring biological markers such as genomic markers, e.g. single nucleotide polymorphisms (SNP), etc. Today, various genomic prediction methods exist and are widely applied, for example, "genomic best linear unbiased prediction" (GBLUP) and ridge regression BLUP (RRBLUP).

GBLUP is a method that utilizes a genomic relationship matrix to estimate the genetic merit of an individual. The genomic relationship matrix is estimated from DNA marker information. The matrix defines the covariance between individuals based on observed similarity at the genomic level, rather than on expected similarity based on pedigree, so that more accurate predictions of merit can be made. GBLUP is also used for the prediction of disease risk and for estimating variance components and genomic heritabilities. RRBLUP is often used to estimate marker effects by ridge regression.

However, existing methods for genomic prediction rely on complete genomic relationship matrices, such as marker-based similarity matrices. Missing data in the relationship matrices can be detrimental, as the lack of this information leads to situations in which predictions cannot be made using routine methods or in which predictions cannot be made at all.

### SUMMARY

The invention provides for an improved method and system for predicting a feature of one or more plants as specified in the independent patent claims. Embodiments of the invention are given in the dependent claims. Embodiments of the present invention can be freely combined with each other if they are not mutually exclusive.

In one aspect, the invention relates to a computer-implemented method for predicting a feature of one or more plants. The method comprises:
- receiving a set of pedigree scores, the pedigree scores being indicative of known genealogical relationships of pairs of plant breeding units over two or more generations, the plant breeding unit pairs comprising pairs of plant breeding units within the same generation and comprising pairs of plant breeding units of different ones of the two or more generations, wherein a plant breeding unit is an individual plant or a group of plants;
- receiving an incomplete set of similarity scores, each similarity score being indicative of observed similarities between the two members of a respective one of the pairs of the plant breeding units, wherein the incomplete set of similarity scores is devoid of similarity scores of at least a sub-set of the plant breeding unit pairs;
- aligning the pedigree scores and the similarity scores of identical plant breeding unit pairs;
- automatically analyzing the aligned pedigree scores and similarity scores for determining associations of the similarity scores and of the pedigree scores, thereby computing a predictive model, the predictive model being adapted to estimate a similarity score as a function of a pedigree score;
- applying the predictive model on pedigree scores of the sub-set of the plant breeding unit pairs for computing missing similarity scores for each of the plant breeding unit pairs of the sub-set;
- creating a complete set of similarity scores from the incomplete set of similarity scores and the computed missing similarity scores; and
- using the complete set of similarity scores for computationally predicting a feature of at least one of the plant breeding units or of an offspring of at least one of the plant breeding units.

For example, the receiving of the pedigree scores and/or of the similarity scores can comprise reading the scores from a local or remote data store, e.g. a file, a directory or a database, receiving the scores via a network interface from a remote data source, e.g. an application program or DBMS, or receiving the scores from a user via a graphical user interface (GUI).

For example, the complete set of similarity scores can be input into a GBLUP or RRBLUP software program or software module for predicting a plant's genetic merit, e.g. in the form of the plant's or plant unit's breeding value.

Embodiments of the invention may have the advantage that the plant's breeding value and other features of interest can be predicted accurately using a standard prediction algorithm even in cases when the available similarity score data set is incomplete, as is often the case in praxis due to the enormous costs often associated with determining certain plant features empirically.

Pedigree records are in many cases abundant. For example, in plant breeding projects, it is essential to document the plant varieties which are crossed in every generation in order to document the project and in order to be able to reproduce the outcome of the breeding project. Hence, pedigree data is often readily available without the need of performing empirical tests. By determining associations between the expensive but incomplete similarity scores and the pedigree data, a predictive model can be obtained which is adapted, according to embodiments of the invention, to infer missing similarity data in non-pedigree-derived similarity score datasets, such as marker-based similarity matrices, for example, based on automatically determined associations of pedigree scores and similarity scores. Hence, as the predictive model is derived from observed associations of similarity scores and pedigree scores in a given set of plant unit pairs, embodiments of the method may flexibly be applied to any type of plant breeding approach and/or on data derived from many different plant species and varieties.

Embodiments of the invention may have the advantage that an abundance of extra information can be provided automatically that can be used for improving prediction quality. Furthermore, embodiments of the invention may allow integrating two different but related types of information (e.g. limited and/or recent molecular marker similarity scores and overall dense pedigree-based similarity scores) for increasing and improving the data basis used for performing a prediction.

According to embodiments, the similarity scores and the pedigree scores of all or most of the plant breeding unit pairs belonging to the latest (youngest) one of the generations are known and are received by the software program configured to perform the method according to embodiments of the invention. However, the similarity scores of all or most of the remaining plant breeding unit pairs belonging to the older ("earlier") ones of the generations are not known and are not received by the software program. For the remaining plant breeding unit pairs of the older ones of the generations only the pedigree scores are known and are received by the software.

This situation is often present in plant breeding programs. Embodiments of the invention may have the advantage that they are particularly suited for allowing to predict features of plants in a plant breeding project even in case the similarity scores of the youngest generation is incomplete and similarity scores of plant breeding unit pairs in all earlier generations are completely unknown.

According to embodiments, the pedigree scores are indicative of known genealogical relationships of all the pairs of plant breeding units over three or more generations.

Embodiments of the invention may have the advantage that pedigree data providing a complete or basically complete genealogic coverage over three or more generations can be used for accurately inferring similarity scores from pedigree scores. The inheritance of a genomic or genetically determined metabolic or phenotypic trait over several generations is a complex process. The process is determined by the quantity and size of chromosomes, the position of the one or more genes causing this trait on these chromosomes, the degree of polyploidy, crossing-over events, the distribution of these genes and alleles in the founder individuals, random events and much more. In general, a large number of generations covered by the pedigree data may increase the accuracy of the predictive model derived from the pedigree data.

An "association" of similarity scores and pedigree scores as used herein refers to any kind of interrelation of the similarity scores and the pedigree scores. According to some embodiments, the determined associations of the similarity scores and of the pedigree scores are correlations, e.g. positive and/or negative correlations. Depending on the embodiment, the trained predictive model implements the learned association explicitly, e.g. in the form of equations, e.g., polynomial equations, or implicitly, e.g. in the form of weights of a neural network.

According to embodiments, the predictive model is a linear or non-linear function that has been fitted on the pedigree scores and the similarity scores such that it returns an estimated similarity score of a plant unit pair in dependence on a pedigree score of the plant breeding unit pair. Preferably, this function is a polynomial function. Preferably, the polynomial function has a polynomial order of preferably 3. However, polynomial functions of another order may also be used in other embodiments.

Embodiments providing the predictive model in the form of a function may have the advantage that the association of pedigree scores and similarity scores is made explicit, thereby enabling a user to review and/or modify the predictive model manually.

According to other embodiments, the predictive model is a trained machine-learning model. The trained machine learning model is a data entity or software entity that has learned during a training phase to estimate a similarity score of a plant unit pair in dependence on a pedigree score of the pair of plant breeding unit pair.

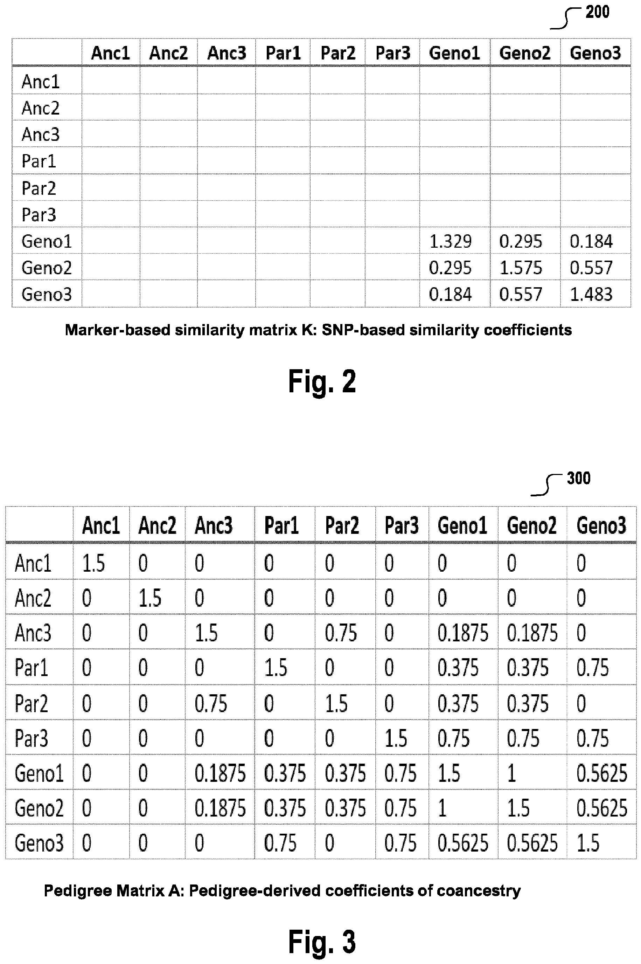

According to embodiments, the method further comprises creating a pedigree score matrix, and using the pedigree score matrix as the set of pedigree scores. For example, a pedigree score matrix "A" as depicted in figure 3 can be created, wherein the value in a cell represents the pedigree score computed for the pair of plant breeding units represented by the column and the row of the matrix "A" identified by the matrix coordinates of this cell.

In addition, or alternatively, the method further comprises creating a similarity score matrix, and using the similarity score matrix as the incomplete set of similarity scores. For example, a marker-based similarity matrix "K" as depicted in figure 2 can be created, wherein the value in a cell represents the similarity score computed for the pair of plant breeding units represented by the column and the row of the matrix "K" identified by the matrix coordinates of this cell.

Embodiments using pedigree score and/or similarity score matrices may have the advantage that the alignment of scores of identical plant breeding unit pairs and the comparison of respective scores can be performed efficiently. Furthermore, the matrix format can be interpreted by both human users and software programs easily. However, other data structures, e.g. vectors or arrays may likely be used for providing and comparing the pedigree and similarity scores.

According to embodiments, the method comprises computing the set of pedigree scores from a genealogical pedigree tree and from predefined scores for different genealogical relationships. For example, a table comprising a list of predefined genealogical relationships such as "parent-child", "full sibs", "half sibs", "double first cousins", "first cousins", etc. can be provided. In this table, each predefined genealogical relation has assigned a predefined pedigree factor. The predefined pedigree factors are used for computing the pedigree scores of the pairs of plant breeding units in accordance with the genealogical relationship of the plant breeding unit pair members.

Embodiments using a set of predefined genealogical relationships for computing the pedigree scores may have the advantage that a pedigree score can be computed quickly for every possible pair of plant breeding units, including pairs having a very complex, multi-generation genealogical relationship.

According to embodiments, the pedigree scores are coefficients of coancestry. Each coefficient of coancestry indicates the probability that one feature (e.g. an allele), derived from the same common ancestor, is identical by descent in two individuals.

All diploid individuals have two alleles (paternal and maternal) at a locus and each parent has 50% chance for transmitting one or the other of these alleles to the offspring. Thus, an allele of a grandmother has probability of 0.5 to be transmitted to her daughter or son and that individual again has probability of 0.5 for being transmitting to the grandchildren. Hence, the probability that two first cousins would have inherited the same allele from the grandmother is 0.0625 (0.5^4).

For example, the coefficient of coancestry, also called the coefficient of consanguinity, between two individuals x and y (f_{xy}) is the probability that two alleles (at the same locus) drawn at random (one from each individual) are identical by descent (Lynch & Walsh 1998).

According to embodiments, the pedigree scores are scores computed as a function of the coefficients of coancestry.

For example, the pedigree scores can be scores computed as Malecot's coancestry coefficients (Malécot, G. 1948, "Les mathématiques de l'hérédité", Paris, Masson & Cie).

According to another example, the pedigree scores are coefficients of (genealogical) relatedness. In the example provided above, the coefficient of coancestry between two individuals x and y is f_{xy}, the coefficients of (genealogical) relatedness r_{xy} between two individuals x, y can be computed as 2f_{xy}.

According to another example, the pedigree scores are inbreeding coefficients. In the example provided above, the coefficient of coancestry between two individuals x and y is f_{xy}, and the inbreeding coefficient of an individual (f) is computed as the coefficient of coancestry of its parents.

According to embodiments, an inbreeding coefficient can be used as a pedigree score as it provides an estimate of the probability that two alleles (or other type of marker) at any given locus are identical by descent (alleles are descendants from a single ancestor and are, thus, identical by descent. Likewise, an inbreeding coefficient can be used as an estimate of the probable proportion of an individual's biological markers (e.g. loci containing genes that are identical by descent).

According to embodiments, the pedigree scores are inbreeding coefficients having been computed using a computational approach that is particularly suited for computing an inbreeding coefficient in plants, e.g. Falconer and Mackay, 1996; Bourdon, 2000). The inbreeding coefficient is both the coefficient of coancestry between the plant unit parents and the coefficient of coancestry of a plant breeding unit to itself.

According to embodiments, a pedigree score matrix is used which comprises the inbreeding coefficient on the diagonal of the pedigree matrix. The other off-diagonal elements of the pedigree matrix refer to the coefficient of coancestry. The "inbreeding coefficient" and the "coefficient of coancestry" both measure the same thing in principle but they are named differently.

According to embodiments of the invention, the pedigree scores are computed based on the initial assumption that founders of the pedigree are unrelated and non-inbred. Under these circumstances, in a diploid species, the coefficient of coancestry is 0.25 between a parent and offspring, their coefficient of (genealogical) relatedness is 0.5 and the offspring of a parent-offspring mating has an inbreeding coefficient of 0.25. However, these numbers may be different for polyploid species and/or for breeding projects using founder plant breeding units which are related.

For example, the pedigree scores are computed as inbreeding coefficients as described in D. S. Falconer and Trudy F. C. Mackay: "Introduction to Quantitative Genetics (4th Edition)", December 05, 1995, chapter "Coancestry or kinship", pages 85 - 88.

According to another example, the pedigree scores are computed as coefficient of coancestry termed "relatedness based on pedigree" as described in the manual of the R package "synbreed", version 0.9-4 dated September 26, 2012, function "kin" on pages 22-24.

According to embodiments, the method further comprises computing each of the similarity scores in the incomplete set of similarity scores as a function of genetic, metabolic, transcription-related, protein-related and/or phenotypic markers of the two plant breeding units comprised in the plant breeding unit pair for which the similarity score is computed. The similarity scores are indicative of a degree of similarity of the markers of the two plant breeding units.

For example, the similarity scores are computed as "marker-based relatedness" as described in the manual of the R package "synbreed", version 0.9-4 dated September 26, 2012, function "kin" on pages 22-24.

According to another example, the similarity scores are computed in accordance with VanRaden P. M., 2008, "Efficient methods to compute genomic predictions", J. Dairy Sci., 91: 4414-4423. The paper also describes the computation of breeding values based on the marker-based similarity scores.

According to a further example, the similarity scores are averaged, haploblock based similarity scores computed as described, for example, in Front Genet. 2018; 9: 364., doi: 10.3389/fgene.2018.00364, PMCID: PMC6127733, "Genomic Prediction of Complex Phenotypes Using Genie Similarity Based Relatedness Matrix", Ning Gao et al. Firstly, in order to build haploblocks in genie regions of various plant species, SNPs were mapped to protein coding genes according to their corresponding physical positions. For each gene, haplotypes were constructed throughout the gene under consideration. Within each haplotype block, allele similarity matrix was constructed by considering the SNP matching pattern between haplotype alleles. Furthermore, the allele similarity matrix was converted into individual similarity matrix. The final marker-based similarity score matrix, referred to as "relatedness matrix", was calculated by averaging the similarity matrices for all haploblocks.

According to embodiments, each of the similarity scores is a marker-based similarity score, in particular a genomic relationship score computed from DNA marker information, or a marker co-occurrence score. For example, the marker-based similarity score can be a numerical value that is a measure of the similarity of two or more compared markers. The numerical value may be a bit value, wherein "1" may represent the absence of the marker in both individuals and "0" may represent the presence of the marker in both individuals. More preferably, the numerical value is a value within a continuous scale which indicates the degree of similarity of the two markers. For example, the similarity of two instances of a marker like "leaf size", or "crop yield" or the edit distance between two compared DNA sequences will typically not be represented as bit value but rather as a numerical value within a value range, e.g. within 0 and 1.

According to some embodiments, the marker is a genetic, metabolic, transcription-related, protein-related, phenotype-related marker and/or breeding value of a plant used as one of the plant breeding units. A genetic marker can be, for example, a gene or a SNP.

According to other embodiments, the marker is an aggregate value derived from genetic, metabolic, transcription-related, protein-related, phenotypic markers and/or or breeding value of a group of plants used as one of the plant breeding units.

According to embodiments, one or more of the plant breeding units respectively consist of a group of plants.

According to some examples, one or more of the plant breeding units can be, for example, a group of plants having the same or a highly similar genotype that is different from the genotype of some or all other ones of the plant groups.

According to other embodiments, one or more of the plant breeding units can be a group of plants belonging to the same cultivar, the cultivar being different from the cultivar to which the plants of some or all of the other plant groups belong to.

It should be noted that a certain cultivar may be used more than once in a multi generation breeding experiment, so the cultivar used as a "breeding unit" may differ from some but not necessary from all other breeding units.

According to some embodiments, the plant breeding units are a group of plants. For each plant breeding unit, the similarity score is computed as an aggregate value of the similarity scores obtained by comparing individuals of the two different plant groups. The aggregation can comprise e.g. computing the geometric or arithmetic mean. For example, the relatedness to/similarity of a group of plants having genotype X in respect to a group of plant having genotype Y can be calculated as the means of the genotype similarity score of all individuals in the plant group having genotype X to the genotype Y. The computation of the pedigree scores for plant groups is even simpler, since each offspring in a biparental cross will share these values.

According to other embodiments, the plant breeding units are individual plants.

In case the plant breeding units are groups of plants, the similarity scores and/or pedigree scores are determined or specified per group of plants.

According to some embodiments described below, a cluster analysis is performed e.g. for grouping the plant breeding units into clusters of similar plant breeding units, whereby the similarity may be determined based on one or more different parameters. Then, the generation of the predictive models and/or the imputation of the missing similarity scores may be performed on a per-cluster basis selectively for plant breeding unit pairs comprised in the same cluster. The genetic, phenotypic, and/or metabolic properties of different plant cultivars used in a breeding project may differ from each other, and also the association of these traits with pedigree-based relatedness may vary. Creating cluster-specific predictive model may have the advantage that the particularities of different populations and cultivars is considered, and the accuracy of the model-based prediction of the missing similarity scores may be increased by generating multiple, cluster-specific predictive models. However, the clustering step is an optional step and it is possible to create the predictive model and to impute missing similarity scores without any clustering. For example, in case the plant breeding units are known or suspected to be the offspring of a single plant variety or in case the totality of plant breeding units considered comprise only a few plant breeding units per plant variety, the clustering step may be omitted without having a significant negative impact on the accuracy of the similarity score imputation.

### Cluster Analysis

According to embodiments, the method further comprises performing a cluster analysis on a base population of plant breeding units, thereby identifying a number n of clusters.

The "base population of plant breeding units" can be, for example, the totality of plant breeding units for which pedigree score data is available and which are or have been used in a plant breeding project.

"Cluster analysis" or "clustering" as used herein is the task of grouping a set of objects in such a way that objects in the same group (called a cluster) are more similar (in some sense) to each other than to those in other groups (clusters).

In the following, different approaches for automatically identifying clusters of plant breeding unit pairs will be described.

### i. Marker-Similarity based Clusters

According to one embodiment, each cluster comprises a sub-set of plant breeding units whose genetic, metabolic, transcription-related, protein-related phenotype-related and/or breeding-related markers are more similar to one another than to respective markers of plant breeding units of other ones of the clusters.

According to some embodiments, the clustering step based on marker-based similarity values is performed on plant breeding unit pairs whose similarity scores are already given. According to another embodiment, the cluster analysis comprises a) computing missing similarity scores for each of the plant breeding unit pairs which do not have assigned a similarity score and b) performing a cluster analysis such that a number n of clusters is identified. Each cluster comprises a sub-set of plant breeding units whose similarity score indicate a similarity above a predefined threshold value.

Step a) may be performed as described for embodiments of the invention and may comprise aligning the pedigree scores and the similarity scores of identical plant breeding unit pairs, automatically analyzing the aligned pedigree scores and similarity scores for determining associations of the similarity scores and of the pedigree scores, thereby computing a preliminary, global predictive model, the preliminary, global predictive model being adapted to estimate a similarity score as a function of a pedigree score; and applying the preliminary, global predictive model on pedigree scores of the sub-set of the plant breeding unit pairs for computing missing similarity scores for each of the plant breeding unit pairs of the sub-set, and creating a complete set of (preliminary) similarity scores from the incomplete set of similarity scores and the computed missing similarity scores.

However, in this embodiment, the complete set of similarity scores and the preliminary global predictive model are computed solely for the purpose of performing a cluster analysis. In some other embodiments, the cluster analysis can take place on the pedigree scores, or use prior information. In some embodiments, at least some of the computed similarity scores are replaced by similarity scores having been re-computed based on cluster-specific predictive models.

### Pedigree-Score based Clusters

According to other embodiments, the cluster analysis is performed on a base population of plant breeding units, thereby identifying a number n of clusters, whereby the clusters are identified such that intra-cluster plant breeding unit have pedigree scores indicating a genealogical relatedness that is higher than the genealogical relatedness of inter-cluster plant breeding unit pairs.

### ii. Diagonal Element and Off-Diagonal Element based Clusters

According to other embodiments, the cluster analysis is performed on a base population of plant breeding units, thereby identifying two clusters: one cluster comprises all pairs of plant breeding units with themselves. This cluster corresponds to the totality of diagonal elements of the pedigree matrix. The other cluster comprises off-diagonal elements of the pedigree matrix.

This clustering approach actually represents a splitting of the pedigree matrix elements into a first cluster of diagonal elements and a second cluster of off-diagonal elements of the matrix.

This may have the advantage that diagonal and off-diagonal elements (and respective plant breeding unit pairs) may be used separately, e.g. for creating at least one predictive model having been trained on the diagonal elements of the pedigree matrix and at least one further predictive model having been trained on the off-diagonal elements. This may allow performing the regression analysis and/or model generation separately for inbreeding coefficients (diagonal pedigree matrix elements) and coancestry coefficients (off-diagonal pedigree matrix elements). Grouping diagonal elements into one or more first clusters and off-diagonal elements into one or more other clusters may be advantageous as the diagonal and off-diagonal elements often may have a different intercept. For example, diagonal values will be generally larger than off-diagonal elements. Hence, the accuracy of cluster-specific models may be increased.

### iii. Clusters based on Prior Information

According to embodiment, apriori information is used for identifying clusters of plant breeding unit pairs. For example, the question which genotypes are to be considered related can be obtained from textbooks and publications or from history data obtained within an organization.

### iv. Clusters based on combinations of two or more clustering approaches

According to embodiments, the cluster analysis is performed on a base population of plant breeding units such that two or more of the above-mentioned clustering approaches i-iv are combined.

For example, a combination of pedigree matrix diagonal (identical genotypes) based clustering approach and a similarity-score based clustering approach can be used: for example, in a first step, marker- similarity based clusters are identified, and in a second step, each cluster is split, where applicable, into one sub-cluster selectively comprising diagonal elements (and respective plant breeding unit pairs) and a second sub-cluster selectively comprising off-diagonal elements (and respective plant breeding unit pairs).

For another example, after performing a similarity-based clustering, n similarity based clusters are obtained. In the next step, each of the n clusters is split into a sub-cluster of pedigree matrix diagonal elements and a second sub-cluster of pedigree matrix off-diagonal elements. Hence, the number of the "finally identified" clusters is maximally 2*n.

Combining two or more clustering approaches may have the advantage that the clusters may be based both on pedigree information and on already available similarity information and hence may more accurately represent the actual similarity of the plant breeding units than clusters derived from only a single data source.

### Computing Cluster-Specific Predictive Models

According to embodiments, the identified clusters are then used for providing cluster-specific predictive models and/or for providing an even more accurate ("refined") complete set of similarity scores as described below.

For each of the number n of identified clusters, the following steps are performed:
- identifying pairs of plant breeding units comprised in this cluster; the identification being performed such that each cluster can comprise breeding unit pairs within and across the different generations;
- receiving pedigree scores for each of the identified pairs;
- receiving similarity scores of at least some of the identified pairs;
- aligning of the pedigree scores and the similarity scores of identical plant breeding unit pairs selectively for the pairs in the cluster;
- performing an automated analysis of the aligned pedigree scores and similarity scores for determining associations of the similarity scores and of the pedigree scores in the cluster, thereby computing a cluster-specific predictive model. The cluster-specific predictive model is adapted to estimate a similarity score as a function of a pedigree score.

Hence, for n different clusters, n different predictive models are obtained. The genetic, phenotypic, and/or metabolic properties of different plant cultivars used in a breeding project may differ from each other, and also the association of these traits with pedigree-based relatedness may vary. Creating cluster-specific predictive model may have the advantage that the particularities of different populations and cultivars is considered, and the accuracy of the model-based prediction of the missing similarity scores may be increased.

According to embodiments, a base population of plant breeding units is used as the founding population of a pedigree tree from which the pedigree scores are derived, wherein the base population comprises at least two genetically distinct groups of plant breeding units.

Embodiments of the invention comprising a creation of cluster-specific predictive models may be particularly useful in the context of a plant breeding project where the founding population comprises two or more different varieties.

The n different predictive models can be used and/or combined differently in order to compute the prediction of the feature such that the knowledge incorporated in each of the n different predictive models is taken into account.

According to some embodiments, the plant breeding pairs of some of the clusters may already have been assigned a similarity score. The method comprises selectively applying the cluster-based predictive models of those clusters comprising plant breeding unit pairs with missing similarity scores in the original data on the plant breeding unit pairs of this cluster for completing the similarity scores of the plant breeding unit pairs of this cluster.

Various approaches exist for integrating the knowledge learned by the multiple cluster-specific predictive models:

### a) Combining intra-cluster similarity scores with preliminary similarity scores provided by a preliminary global predictive model

According to embodiments, the complete set of similarity scores is computed as described above as a set of preliminary similarity scores whereby a single predictive model (computed as described above without clustering) is used for computing the complete set of preliminary similarity scores. The method further comprises:
- applying the cluster-specific predictive models on pedigree scores of the sub-set of the plant breeding unit pairs of the one of the clusters from which the cluster-specific predictive model was derived for computing missing similarity scores for intra-cluster plant breeding unit pairs of the cluster;
- supplementing the received incomplete set of similarity scores with the similarity scores computed for the intra-cluster plant breeding unit pairs of the one or more clusters, thereby providing an intermediate incomplete set of similarity scores, the intermediate incomplete set of similarity scores being devoid of similarity scores of at least some of the inter-cluster plant breeding unit pairs;
- supplementing the intermediate incomplete set of similarity scores by using the preliminary similarity scores similarity scores as the missing similarity scores of the inter-cluster plant breeding unit pairs, thereby providing a refined complete set of similarity scores; and
- using the refined complete set of similarity scores for performing the computational prediction of the feature.

In this approach, the cluster-specific predictive models are used for computing missing similarity scores for intra-cluster plant breeding unit pairs. For the missing similarity scores for inter-cluster plant breeding unit pairs (i.e., pairs of plant breeding units whose two members belong to two different clusters), the preliminary global similarity scores computed by the preliminary predictive model are used. Hence, the preliminary similarity scores may be used as a basis for a subsequent cluster analysis and/or for providing the inter-cluster plant breeding unit similarity scores, but many of these preliminary similarity scores may not be used for performing the prediction of the feature.

### b) Using originally received similarity scores and intra-cluster similarity scores for generating a global predictive model

According to an alternative approach, the original set of similarity scores is supplemented with the intra-cluster plant breeding unit pair similarity scores computed by the different cluster-specific predictive models, thereby creating an intermediate set of similarity scores (e.g. an intermediate similarity score matrix) which is still not complete, but is more complete than the originally received set of similarity scores. Then, a final global predictive model is computed based on the intermediate set of similarity scores and the respective pedigree scores as described herein for embodiments of the invention. The final global predictive model is used for computing the missing inter-cluster plant breeding unit pairs, thereby providing a complete set of similarity scores which assigns each intra-cluster plant breeding pair and each inter-cluster plant breeding pair a respective similarity score value.

For example, this approach can be implemented by a method comprising:
- applying the cluster-specific predictive models on pedigree scores of the sub-set of the plant breeding unit pairs of the one of the clusters from which the cluster-specific predictive model was derived for computing missing similarity scores for intra-cluster plant breeding unit pairs of the cluster;
- supplementing the received incomplete set of similarity scores with the similarity scores computed for the intra-cluster plant breeding unit pairs of the one or more clusters, thereby providing an intermediate incomplete set of similarity scores, the intermediate incomplete set of similarity scores being devoid of similarity scores of at least some inter-cluster plant breeding unit pairs;
performing the method comprising score alignment, analysis and predictive model computation as described herein for embodiments of the invention, thereby using the intermediate incomplete set of similarity scores as the received incomplete set of similarity scores to be aligned and analyzed; the predictive model is computed by analyzing the aligned pedigree scores and the similarity scores of the intermediate incomplete set of similarity scores; the computed predictive model (which incorporates the intra-cluster similarity scores computed by the cluster-specific predictive models and hence integrates the "knowledge" of the cluster-specific models) is applied on the pedigree scores of inter-cluster plant breeding unit pairs for creating the complete set of similarity scores that is used for computationally predicting the feature.

### c) Further approaches for computing a global predictive model

According to some embodiments, the method further comprises combining the cluster-specific predictive models of all clusters into a global predictive model.

For example, the cluster-specific predictive models can be polynomial equations and the combination of the cluster-specific predictive model can be a combination of multiple polynomial equations into a single polynomial equation. Alternatively, the cluster-specific predictive models can be used for computing similarity scores for intra-cluster plant breeding unit pairs lacking a similarity score and then computing a global predictive model based on the originally provided similarity scores and on the intra-cluster similarity scores. The totality of originally received similarity scores of some plant breeding unit pairs and the later computed similarity scores of the intra-cluster plant breeding unit pairs is aligned with the respective pedigree scores and used as a basis for creating the global predictive model, thereby integrating the "knowledge" of the cluster-specific predictive model into the global predictive model. Finally, the global predictive model is applied on pedigree scores of a sub-set of the plant breeding unit pairs which do not yet have assigned a similarity score for computing the missing similarity scores. Hence, according to this approach, similarity scores are placed in the designated table or matrix in the following order: first the known/originally received similarity scores are added. Then, if cluster-specific predictive models were generated and used for computing similarity scores for intra-cluster plant breeding unit pairs, these intra-cluster plant breeding unit pair similarity scores are placed into the table or matrix.

And finally, according to embodiments of the invention, a final global predictive model may be computed based on the data content of this table or matrix and the associated pedigree scores. The final global predictive model is used for computing inter-cluster plant breeding unit pair similarity scores, whereby the inter-cluster plant breeding unit pair similarity scores are added to the table or matrix for providing a complete table or matrix of similarity scores.

A multi-step, cluster-based approach may have the advantage that differences between marker-based and pedigree-based estimates that occur due to the history of selection on the breeding material may be considered.

Depending on the type of predictive model used, different "ensemble" techniques for combining multiple predictive models into a global model may be used. For example, the same sort of classifier can be combined using boosting (using e.g. Adaboost) and bagging (using e.g. random forests). AdaBoost, short for Adaptive Boosting, is a machine learning meta-algorithm formulated by Yoav Freund and Robert Schapire. It can be used to improve performance of learning algorithms by combining the output of multiple learning algorithms ('weak learners') into a weighted sum that represents the final output of the boosted classifier.

According to embodiments, the predicted feature is a breeding value of one or more of the plant breeding units. For example, the breeding value can be computed by using genomic best linear unbiased prediction (GBLUP) or ridge regression BLUP (RRBLUP) that is applied on the completed similarity score matrix.

According to other embodiments, the predicted feature is an identifier of one or more of the plant breeding units having the highest likelihood of comprising a favorable genomic, metabolic, or phenotypic marker.

According to embodiments, the predicted feature is an identifier of one or more of the plant breeding units having the highest likelihood of comprising an undesired genomic, metabolic, or phenotypic marker.

According to embodiments, the predicted feature is an identifier of at least one plant breeding unit pair comprising a favorable combination of genomic, metabolic, or phenotypic markers.

According to embodiments, the predicted feature is an identifier of at least one plant breeding unit pair comprising an undesired combination of genomic, metabolic, or phenotypic markers.

According to embodiments, the predicted feature is the likelihood of occurrence of a favorable or of an undesired genomic, metabolic, or phenotypic marker in an offspring of two of the plant breeding units.

In a further aspect, the invention relates to a computer program comprising computer-interpretable instructions which, when executed by a processor, cause the processor to perform a method according to any one of the embodiments described herein. The computer program can be provided in the form of a computer program product embodied in a computer-readable storage medium.

In a further aspect, the invention relates to a method for conducting a plant breeding project, the method comprising:
- providing a group of candidate plant breeding units, wherein a candidate plant breeding unit is an individual plant or a group of plants potentially to be used in the plant breeding project, wherein a known genealogical relationship of pairs of the candidate plant breeding units over two or more generations is available;
- performing the method according to anyone of the method for computationally predicting a feature of at least one of the candidate plant breeding units or of an offspring of at least one of the plant breeding units described herein for embodiments and examples of the invention; the candidate plant breeding units are used as the plant breeding units whose pedigree scores and incomplete set of similarity scores are received; the feature is indicative of whether the at least one candidate breeding unit comprises a favorable genomic, metabolic, or phenotypic marker and/or a favorable breeding value;
- selecting one or more of the candidate breeding units in dependence on the at least one predicted feature; and
- selectively using the selected one or more candidate breeding units for generating offspring in the plant breeding project.

In a further aspect, the invention relates to a computer-system configured for predicting a feature of one or more plants. The computer system comprises one or more processors and a volatile or non-volatile storage medium. The storage medium comprises:
- a set of pedigree scores, the pedigree scores being indicative of known genealogical relationships of pairs of plant breeding units over two or more generations, the plant breeding unit pairs comprising pairs of plant breeding units within the same generation and comprising pairs of plant breeding units of different ones of the two or more generations, wherein a plant breeding unit is an individual plant or a group of plants;
- an incomplete set of similarity scores, each similarity score being indicative of observed similarities between the two members of a respective one of the pairs of the plant breeding units, wherein the incomplete set of similarity scores is devoid of similarity scores of at least a sub-set of the plant breeding unit pairs; and
- a software.

For example, the software can be an application program or a set of two or more application programs. The software can be installed locally on a single computer system or can be installed as a distributed software application, e.g. a cloud service, on multiple interconnected computer systems.

The software comprises computer-interpretable instructions which, when executed by the one or more processors, cause the processors to perform a method comprising:
- aligning the pedigree scores and the similarity scores of identical plant breeding unit pairs;
- analyzing the aligned pedigree scores and similarity scores for determining associations of the similarity scores and of the pedigree scores, thereby computing a predictive model, the predictive model being adapted to estimate a similarity score as a function of a pedigree score;

- applying the predictive model on pedigree scores of the sub-set of the plant breeding unit pairs for computing missing similarity scores for each of the plant breeding unit pairs of the sub-set;
- creating a complete set of similarity scores from the incomplete set of similarity scores and the computed missing similarity scores; and
- using the complete set of similarity scores for computationally predicting a feature of at least one of the plant breeding units or of an offspring of at least one of the plant breeding units.

A "pedigree score" as used herein is a numerical value indicating the similarity of two compared items (plant breeding units) which is derived from and indicative of the proximity of the genealogical relationship of the two items. For example, a pedigree score is a numerical value derived by relationship records and/or parentage records, either traced back or collected through time. A pedigree-score can be a numerical value derived from shared parentage or shared ancestry, or lack thereof. For example, a pedigree score matrix can consist of "coefficients of coancestry" or numerical values derived therefrom.

A "similarity score" as used herein is a numerical value indicating the similarity of two compared items (plant breeding units) which is derived from the similarity of one or more observed or empirically determined properties of the two compared items (e.g. plant breeding units). For example, the similarity score can be indicative of the similarity of two items in respect to one or more genomic, metabolic, phenotypic or other type of marker. A "similarity score" is not derived from pedigree data, but rather from directly observed or measured features of the two compared items. Hence, a "similarity score" is a pedigree-agnostic numerical value. For example, a similarity score can be derived in an in-situ experiment, e.g. by sampling the individuals or families used as plant breeding unit molecularly, phenotypically, etc. Hence, a similarity score is obtained via direct observations and sampling in the populations themselves, rather than derived from their known or unknown relatedness via shared parentage or shared ancestry.

For example, a pedigree score can be described as a "similarity by descent" score and a "similarity score" can be described as a "non-pedigree-based, similarity by state" score.

A "predictive model" as used herein is a set of parameter values, a data structure and/or an executable software program or function which adapted to perform a prediction. For example, the predictive model can be created automatically or semi-automatically in a training phase using a machine-learning technology. The predictive model can be the result of a regression analysis or of another form of data analysis adapted to identify associations and interdependencies between two parameters, i.e., similarity score and pedigree scores. For example, the predictive model can be a trained neural network (NN), a trained support vector machine, etc. The machine-learning technology can use a regression analysis or another data analysis technique for determining associations of the pedigree scores and the similarity scores of aligned plant breeding unit pairs.

The term "Machine learning (ML)" as used herein refers to the study, development or use of a computer algorithm that can be used to extract useful information from training data sets by building probabilistic models (referred to as machine learning models or "predictive models") in an automated way. Machine learning algorithms build a mathematical model based on sample data, known as "training data", in order to make predictions or decisions without being explicitly programmed to perform the task. The machine learning may be performed using a learning algorithm such as supervised or unsupervised learning, reinforcement algorithm, self-learning, etc. The machine learning may be based on various techniques such as clustering, classification, linear regression, support vector machines, neural networks, regression analysis etc. A "model" or "predictive model" may for example be a data structure or program such as a neural network, a support vector machine, a decision tree, a Bayesian network, a polynomial function etc. or parts thereof adapted to perform a predictive task. The model is adapted to predict an unknown value (e.g. a similarity score) from other, known values (e.g. a pedigree score). For example, the ML-model can be a predictive model that has learned to perform a predictive task such as classification or regression. Classification is the problem of predicting a discrete class label output for an input, e.g. a test image or part thereof. Regression is the problem of predicting a continuous quantity output for an input.

As will be appreciated by one skilled in the art, aspects of the present invention may be embodied as an apparatus, method, computer program or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon. A computer program comprises the computer executable code or "program instructions".

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Generally, the program instructions can be executed on one processor or on several processors. In the case of multiple processors, they can be distributed over several different entities like clients, servers etc. Each processor could execute a portion of the instructions intended for that entity. Thus, when referring to a system or process involving multiple entities, the computer program or program instructions are understood to be adapted to be executed by a processor associated or related to the respective entity.

Aspects of the present invention are described with reference to flowchart illustrations and/or block diagrams of methods, apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

In view of the wide variety of permutations to the embodiments described herein, this detailed description is intended to be illustrative only, and should not be taken as limiting the scope of the invention. What is claimed as the invention, therefore, is all such modifications as may come within the scope of the following claims and equivalents thereto. Therefore, the specification and drawings are to be regarded in an illustrative rather than a restrictive sense.

### BRIEF DESCRIPTION OF THE DRAWINGS

In the following, only exemplary forms of the invention are explained in more detail, whereby reference is made to the drawings in which they are contained. They show:
- Figure 1: a flowchart of a method for predicting a feature of a plant breeding unit using incomplete similarity scores and pedigree scores;
- Figure 2: shows an example of an incomplete similarity score matrix;
- Figure 3: shows an example of a pedigree score matrix;
- Figure 4: shows an example of a complete similarity score matrix;
- Figure 5: shows a block diagram of a computer system used for predicting a feature of a plant breeding unit using incomplete similarity scores and pedigree scores;
- Figure 6: is a scatterplot showing similarity scores and pedigree scores and a polynomial curve fitted to the scores without applying cluster analysis; and
- Figure 7: is a scatterplot showing similarity scores and pedigree scores and a polynomial curve fitted to clusters of scores.

### DETAILED DESCRIPTION

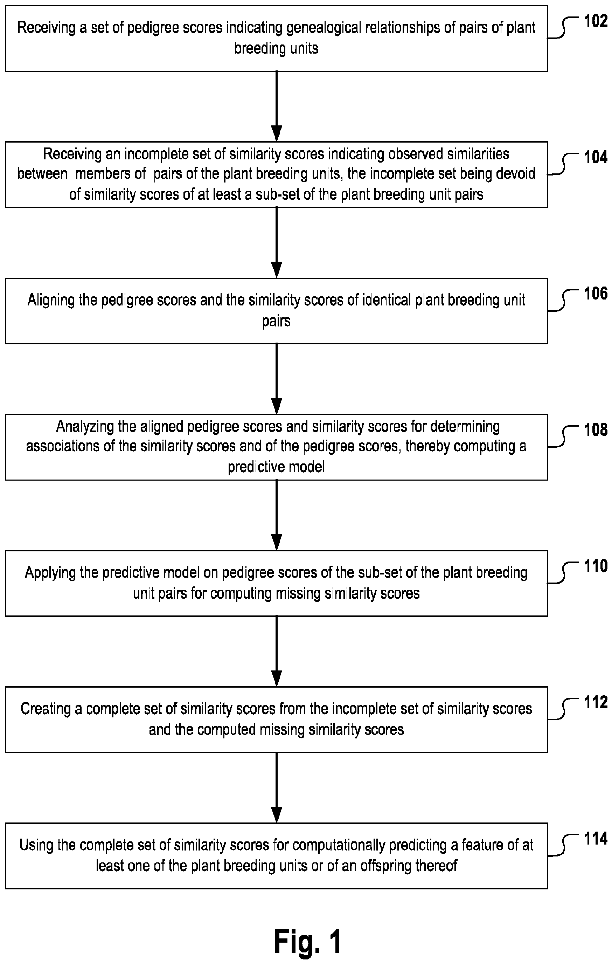

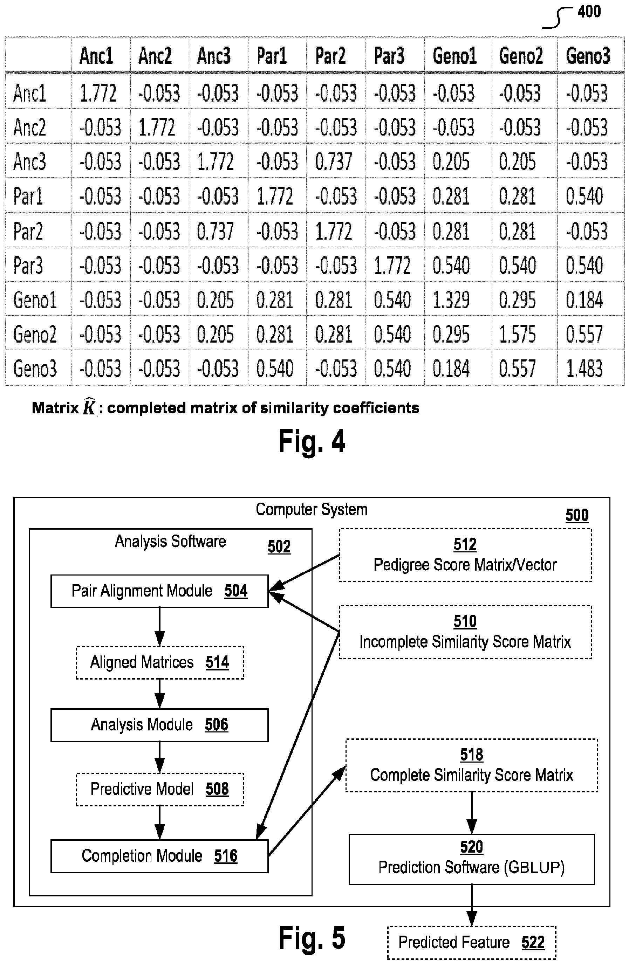

**Figure 1** shows a flow chart of an example of a method for predicting a feature of a plant breeding unit. The method can be executed, for example, by a system 500 depicted in figure 5. For example, the method can be executed by one or more components of the system, e.g. a software 502 for generating a predictive model and a complete similarity score matrix and a software 520 for predicting the feature of the plant breeding unit.

In the following, the method according to figure 1 will be described by making reference to the system of figure 5. However, the method can likewise be performed by other data processing systems.

The examples described herein may allow imputing missing data points in a non-pedigree-derived similarity score dataset, such as a marker-based similarity score matrix (which is sometimes used to estimate kinship and hence is sometimes also referred to as "kinship matrix" although no pedigree information was used for constructing this matrix). The imputed data is computed from a pedigree score dataset. Missing similarity data in this context can be detrimental, as the lack of this information leads to situations in which predictions cannot be made using routine methods for genomic prediction.

In the example described here, genotypic information of a set of individuals in recombination cycle D was used together with a portion of the genotypic information from the recombination cycles of the parental generation cycles (A, B, and C - though many parents were ungenotyped). Based on this data, the incomplete similarity score matrix was computed based on molecular marker data. Additionally, pedigree score data was obtained for those genotypes from recombination cycle D. The data also contains parental information for several generations (potentially around 5 generations or less).

In a first step 102, a computer system 500 or the analysis software 502 receives the above-mentioned set of pedigree scores 512. For example, the pedigree scores can be read in the form of a pedigree score matrix 300, 512 as depicted, for example in figure 3, from a storage medium of the computer system 500. The pedigree scores are indicative of known genealogical relationships of pairs of plant breeding units over two or more generations. For example, the plant breeding units "Anc1", "Anc2" and "Anc3" may constitute the founder generation. The plant breeding units "Par1", "Par2" and "Par3" may constitute the parent generation. And the "Geno1", "Geno2" and "Geno3" plant breeding units may constitute the youngest generation of plant breeding units. The pedigree scores can be received already in the pedigree score matrix or vector form or may be received in another format and then converted in a pedigree score matrix (referred to as ***A*)*.*** In the example described here, the pedigree scores were computed as coefficients of coancestry "f" as described in D. S. Falconer and Trudy F. C. Mackay: "Introduction to Quantitative Genetics (4th Edition)", December 05, 1995, chapter "Coancestry or kinship", pages 85 - 88.

The aim of the plant breeding project used for this example was to identify a sub-set of the plant breeding units of the youngest generation and a further sub-set of plant breeding units of the "parent" or "founder" generation (or genetically equivalent plants) in order to generate offspring having one or more desirable traits. For example, a big leaf size and high heat stress tolerance may be considered desirable traits. The leaf size and heat stress tolerance in the youngest generation may already be close to optimum, but in order to get rid of an undesired trait that is common in the youngest generation, it may be desirable to cross selected ones of the plants of the youngest generation with selected ones of the plants of the parent or founder generations (or genetically equivalent plants) in order to obtain plants having the desired properties and at the same time being free of the undesired property. The problem may be that marker-based similarity information, e.g. information on SNPs known to correlate with the desired traits, may only be available for the youngest generation "Geno1-3", not for the parent or founder generation.

The matrix depicted in Fig. 3 is for illustration purposes only and is much smaller than the pedigree score matrix to be used in the context of a typical plant breeding project covering many hundreds or even thousands of plant breeding units.

Next in step 104, the system 500 receives an incomplete set of similarity scores 200, 510. An example of an incomplete similarity score matrix 200 is presented in figure 2. The set of similarity scores is incomplete, because for some plant breeding unit pairs (e.g. Anc1-Anc1, Anc1-Anc3, Anc1-Par1, Anc2-Geno3), no similarity score is available. As marker-based information, e.g. information regarding SNPs or other molecular traits used for computing the similarity scores for plant breeding unit pairs within the youngest generation, is not available for the founder generation and for the parent generation, any pair of plant breeding units comprising a plant breeding unit of the founder or parent generation will not comprise a marker-based similarity score.

Next in step 106, the system having received the pedigree scores and incomplete similarity scores aligns the pedigree scores and the similarity scores of identical plant breeding unit pairs. For example, the pedigree score matrix and the incomplete similarity score matrix may be received as or transformed into symmetrical matrices that can be aligned to each other easily. Alternatively, the pedigree scores and the similarity scores are aligned in the form of score vectors. In this case, the matrix alignment is an illustration of the ones of the scores which are aligned to each other while in fact the alignment process is performed based on an alignment of pedigree score vectors and similarity score vectors.

Next in step 108, the system automatically analyzes the aligned pedigree scores and similarity scores for determining associations of the similarity scores and of the pedigree scores for computing a predictive model 508 which is able to computationally estimate a similarity score as a function of a pedigree score.

In order to create the predictive model, the scores of those plant breeding unit pairs for which all pairwise information for both ***K*** (similarity scores in the form of marker-based similarity coefficients) and ***A*** (pedigree scores in the form of coefficients of coancestry) exists, are analyzed. In the specific example depicted in figures 2-4, that means that only the information for Geno1, Geno2, and Geno3 is used for the creation of the predictive model. In this example, the predictive model is a polynomial function of order 3 that was fitted during a regression analysis to the aligned similarity and pedigree score data. Alternatively, other methods for computationally creating predictive models can also be employed, for example, higher or lower order polynomials, linear regression, splines, or ARIMA-based fitting.

Next in step 110, the system, the system 500 applies the predictive model (the polynomial function of order 3 created in the previous step) on the pedigree scores of at least the sub-set of the plant breeding unit pairs currently lacking a similarity score for computing the missing similarity scores.

Then in step 112, a complete similarity score matrix as depicted in figure 4 is generated by combining the received similarity scores with the similarity scores computed by the predictive model.

Next in step 114, the system inputs the complete set of similarity scores into a prediction software 520 that is adapted to computationally predict a feature 522 of a plant breeding unit or of an offspring thereof based on the complete set of similarity scores. For example, the prediction software 520 can use the GBLUP algorithm for computing a predictive value based on the complete similarity score matrix 400.

According to some examples, the pedigree score matrix is transformed into a pedigree score vector x and the incomplete similarity score matrix is transformed into an incomplete similarity score vector y. The alignment of scores and the score analysis for creating the predictive model is performed on vectors rather than a matrix structure. Transforming the matrices into vectors may further increase the performance as some programs for statistical (regression) analysis expect to receive two or more data vectors as input.

For example, the transformation of the matrix into a vector can be performed as follows:
1. Start with a matrix, e.g. a similarity score matrix

| | Geno1 | Geno2 | Geno3 | Geno4 |
|---|---|---|---|---|
| Geno1 | 1.5 | 0.4 | 0.9 | 0.7 |
| Geno2 | 0.4 | 1.5 | 0.5 | 0.4 |
| Geno3 | 0.9 | 0.5 | 1.5 | 0.4 |
| Geno4 | 0.7 | 0.4 | 0.4 | 1.5 |

2. Remove either the upper or lower triangle of the matrix, which doesn't matter since it is symmetrical around the diagonal

| | Geno1 | Geno2 | Geno3 | Geno4 |
|---|---|---|---|---|
| Geno1 | 1.5 | | | |
| Geno2 | 0.4 | 1.5 | | |
| Geno3 | 0.9 | 0.5 | 1.5 | |
| Geno4 | 0.7 | 0.4 | 0.4 | 1.5 |

3. Taking just the columns and stacking them; (one could also stack the rows; this doesn't matter as long as one is consistent when applying them to the two matrices, the pedigree score matrix derived from a pedigree dataset and the similarity score matrix derived from marker data).

This leads to a table of three columns: Genotype 1, Genotype 2, and their marker-based similarity score

| | | |
|---|---|---|
| Geno1 | Geno1 | 1.5 |
| Geno2 | Geno1 | 0.4 |
| Geno3 | Geno1 | 0.9 |
| Geno4 | Geno1 | 0.7 |
| Geno2 | Geno2 | 1.5 |
| Geno3 | Geno2 | 0.5 |
| Geno4 | Geno2 | 0.4 |
| Geno3 | Geno3 | 1.5 |
| Geno4 | Geno3 | 0.4 |
| Geno4 | Geno4 | 1.5 |

**Figure 2** shows an example of an incomplete similarity score matrix 200. The similarity scores have been derived from molecular marker estimated similarity coefficients (sometimes also referred to as "kinship coefficients" or "kinship coefficient estimates"). Only recent individuals ("Geno1-3") have marker-based similarity scores.

**Figure 3** shows an example of a pedigree score matrix 300. The pedigree scores in the matrix 300 have been computed as pedigree-derived coefficients of coancestry. Depending on the depth of the pedigree information, this matrix may be relatively sparse (here: comprise many cells filled with "0"), but will nevertheless comprise more cells filled with a score than the similarity score matrix 200. Founder parents "Anc1-3" are assumed to be unrelated.

**Figure 4** shows an example of a complete similarity score matrix 400. The matrix 400 represents a semi-imputed matrix of the similarity scores between individual pairs of plant breeding units. Depending on the depth of the pedigree and genotype score information, cells may contain the same value (but unlike the pedigree derived data, can comprise a negative value.

**Figure 5** shows a block diagram of a computer system 500 used for predicting a feature of a plant breeding unit using incomplete similarity scores and pedigree scores. The computer system can be, for example, a standard computer system, a server computer system, a portable computer system, a monolithic or a distributed computer system, e.g. a cloud computer system. The computer system may comprise a prediction software 520 and an analysis software 502 with multiple sub-modules 504, 506, 516. However, it is also possible that the functionalities provided by the software programs 520, 502 and/or modules 504, 506 and 516 are integrated into a single software programs or are distributed over three or more different software programs.

In a first step, the analysis software 502 reads an incomplete similarity score matrix 510 from a local or remote data store. The matrix 510 is a similarity score dataset in which data is missing regarding the marker-based similarity between members of pairs of plant breeding units, e.g. individual plants, genotypes or cultivars. In addition, pedigree scores 512 available for the above-mentioned pairs of plant breeding units is received by the software 502. In the ideal case, the pedigree score data set is deep, meaning it cover several previous generations (here: "Anc" and "Par"). The pedigree score data is received or transformed into a pedigree score matrix (referred to as ***A)*** as depicted, for example, in figure 3.

The matrix comprising the incomplete similarity score data set is referred to as y and the matrix comprising the pedigree scores is referred to as x. The software 502 comprises an alignment module 504 configured to align (or map) pedigree scores to similarity scores (if any) assigned to the same pair of plant breeding units. For example, the alignment of matrices can be implemented as an alignment of vectors.

An analysis module is adapted to analyze the association of the aligned pedigree scores and similarity scores for automatically creating a predictive model that is adapted to predict a similarity score from a given pedigree score. For example, the association module may perform a regression analysis for fitting a polynomial model to the aligned scores (having been placed in the proper format for the analysis module). For example, a polynomial function of order 3 may be fitted by regressing the incomplete similarity score matrix y on the pedigree score matrix x.

Preferably, the regressing of the incomplete similarity score matrix y on the pedigree score matrix is implemented based on a score vector alignment and regression. For example, the regression process may comprise a) representing the pedigree score matrix x as a vector vx (see description of figure 1), representing the similarity score matrix y as vector vy, aligning the scores of vx and vy, and performing the regression on the two aligned vectors.

Then, the association module 506 outputs the created predictive model 508.

A further module of the analysis software 502, the completion module, 516, applies the predictive model 508 on the pedigree scores 512 for computing the missing similarity scores. The empty cells of matrix 510 are filled with the newly computed similarity scores and a complete similarity score matrix 518 is provided. A concrete example of this matrix 518 is depicted in figure 4. The completed similarity score matrix 518 is output to a prediction software 520, e.g. a software implementing the GBLUP algorithm.

The prediction software computes a prediction of one or more features 522 of one or more plant breeding units or the offspring thereof based on the completed similarity score matrix 518. The feature 522 is output to a user, e.g. via a GUI or a printer. The feature can be, for example, a breeding value, a predicted likelihood of the presence of one or more desired or undesired genotypic, metabolic and/or phenotypic traits, or the like.

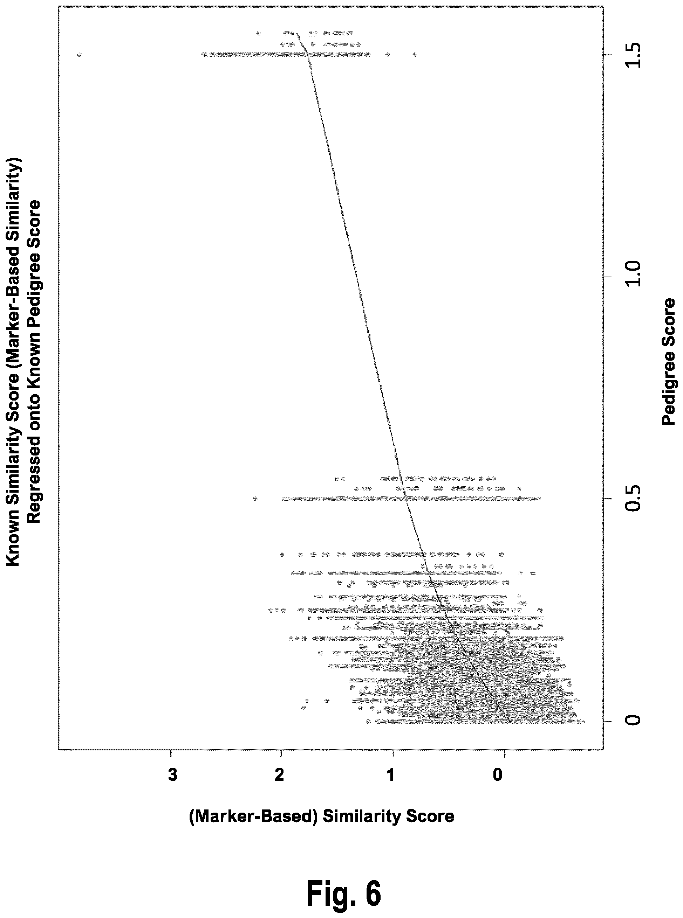

**Figure 6** is a scatter plot showing data points based on similarity scores and pedigree scores and a polynomial curve. The curve was created by fitting a polynomial function to the totality of received and aligned (incomplete) similarity and pedigree scores 514. No clustering of plant breeding units and respective scores was performed.

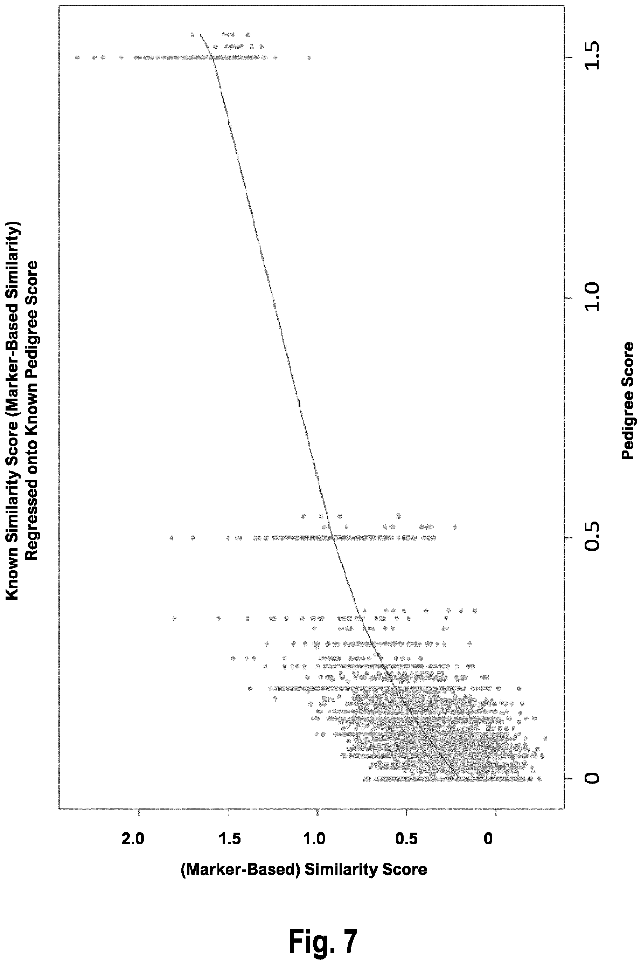

**Figure 7** is a scatter plot showing similarity scores and pedigree scores and a polynomial curve fitted to clusters of scores.

To obtain the curve of figure 7, a cluster analysis was performed on the plant breeding units.

According to some examples, the clustering of plant breeding units is performed based on biological markers of these plant breeding units (e.g. the biological markers used for computing the incomplete similarity scores). According to another example, the clustering is performed on the pedigree scores.

According to some examples, the clustering is performed after the steps 102-112 have been performed on the totality of originally received and imputed similarity scores. For example, the clustering algorithm k-means or a similar clustering algorithm can be used for identifying the clusters. According to some further examples, the clustering can also be performed semi-automatically based on prior information related to population structure, geographic structure, or any other information being characteristic for the plant breeding units used in a plant breeding project.

In the given example, the cluster analysis was performed on the totality of originally received and imputed similarity scores. The cluster analysis identified nine different clusters of plant breeding units. Pairs of plant breeding units belonging to the same cluster formed clusters of pairs of plant breeding units.

Then, an alignment of the similarity scores and pedigree scores and the creation of a predictive model as described e.g. with reference to steps 106-108 was performed on a per-cluster basis on the plant breeding unit pairs belonging to a particular one of the nine clusters.

This may have the advantage that cluster-specific predictive models may be able to describe potentially different score relationships of plant breeding unit pairs among and between different clusters. One example of these intra-cluster model fits is shown in Figure 7. The similarity score ("kinship coefficient") between the ancestral plant breeding units found to belong to the same cluster was imputed using only the "ancestral-cluster specific predictive model". The clustering of plant breeding units for creating and using cluster-specific predictive models was implemented to account for differences between marker-based and pedigree-based estimates that occur due to the history of selection on this breeding material.

For example, the determination of n different clusters during cluster analysis may be used to split the score data into n (complete or incomplete) vectors comprising the similarity score values of the respective clusters and n further vectors comprising the pedigree scores of the plant breeding units of the clusters. Curve fitting and regression analysis is performed for each of the n clusters and respective vector pairs separately for creating n different predictive models. This may provide a greater level of detail that allows a better fit of data that deviate from pedigree relatedness due to selection. The combined similarity score matrix is created by using the n different predictive models for computing the missing similarity scores (in case a cluster does not comprise missing similarity score, executing the respective predictive model may not be necessary).

In a final, optional step, a global predictive model may be created by regressing the - meanwhile completed - set of similarity scores to the pedigree scores of the whole data set. Hence, a single predictive model is obtained that integrates the cluster-specific knowledge on the relationship of pedigree scores and similarity scores.

According to one example, missing similarity score values are computed and placed in the designated table or matrix at the end of the procedure in the following order: First the received and already existing similarity scores are added to the matrix. Then, if the plant breeding units and respective score values were clustered, the clustered predicted pairwise similarity scores are placed into the matrix, thereby providing a completed matrix of similarity scores. Then, a global predictive model is obtained by analyzing the completed similarity score matrix and the pedigree score matrix aligned to the completed similarity score matrix. For example, the analysis may be based on fitting a polynomial curve, by applying a machine learning algorithm or the like. And finally, the global predictive model is applied on the pedigree data of all plant breeding unit pairs originally missing a similarity value to obtain final similarity scores. The combination of the originally received similarity scores and the similarity scores computed by the global predictive model is used as the final, completed similarity score matrix. For example, this final, completed similarity score matrix can be input to a genomic prediction software for predicting a feature of a plant breeding unit.

A comparison of the plot depicted in figures 6 and 7 reveals that the predictive model obtained with the clustering-based approach (here: the fitted curve of figure 7) can be highly similar to the predictive model generated in a non-clustering-based approach (here: the fitted curve of figure 6). So the effect of the optional clustering step on the accuracy of the predictive model may depend on the properties of the totality of the plant breeding units whose similarity scores are to be completed computationally.

### List of Reference Numerals

- 102-114: steps
- 200: incomplete similarity score matrix
- 300: pedigree score matrix
- 400: complete similarity score matrix
- 500: computer system
- 502: analysis software
- 504: score alignment module
- 506: score association module
- 508: predictive model
- 510: incomplete similarity score matrix
- 512: pedigree score matrix
- 514: aligned matrices 200, 300
- 516: similarity score completion module
- 518: completed similarity score matrix
- 520: prediction software

## Claims

1. A computer-implemented method for predicting a feature of one or more plants, the method comprising:
- receiving (102) a set of pedigree scores (300, 512), the pedigree scores being indicative of known genealogical relationships of pairs of plant breeding units over two or more generations, the plant breeding unit pairs comprising pairs of plant breeding units within the same generation and comprising pairs of plant breeding units of different ones of the two or more generations, wherein a plant breeding unit is an individual plant or a group of plants;
- receiving (104) an incomplete set of similarity scores (200, 510), each similarity score being indicative of observed similarities between the two members of a respective one of the pairs of the plant breeding units, wherein the incomplete set of similarity scores is devoid of similarity scores of at least a sub-set of the plant breeding unit pairs;
- aligning (106) the pedigree scores and the similarity scores of identical plant breeding unit pairs;
- automatically analyzing (108) the aligned pedigree scores and similarity scores for determining associations of the similarity scores and of the pedigree scores, thereby computing a predictive model (508), the predictive model being adapted to estimate a similarity score as a function of a pedigree score;
- applying (110) the predictive model on pedigree scores of the sub-set of the plant breeding unit pairs for computing missing similarity scores for each of the plant breeding unit pairs of the sub-set;
- creating (112) a complete set of similarity scores (400, 518) from the incomplete set of similarity scores and the computed missing similarity scores; and
- using (114) the complete set of similarity scores for computationally predicting a feature (522) of at least one of the plant breeding units or of an offspring of at least one of the plant breeding units.

2. The computer-implemented method of claim 1, wherein the pedigree scores are indicative of known genealogical relationships of all the pairs of plant breeding units over three or more generations.

3. The computer-implemented method of any one of the previous claims, wherein the predictive model is selected from a group comprising:
- a linear or non-linear function that has been fitted on the pedigree scores and the similarity scores such that it returns an estimated similarity score of a plant unit pair in dependence on a pedigree score of the plant breeding unit pair, the function being preferably a polynomial function having a polynomial order preferably of 3;
- a trained machine-learning model, the trained machine learning model having learned during a training phase to estimate a similarity score of a plant unit pair in dependence on a pedigree score of the pair of plant breeding unit pair.

4. The computer-implemented method of any one of the previous claims, further comprising:
- creating a pedigree score matrix, and using the pedigree score matrix as the set of pedigree scores; and/or
- creating a similarity score matrix, and using the similarity score matrix as the incomplete set of similarity scores.

5. The computer-implemented method of any one of the previous claims, further comprising:
- computing the set of pedigree scores from a genealogical pedigree tree and from predefined scores for different genealogical relationships.

6. The computer-implemented method of any one of the previous claims, the pedigree scores being selected from a group comprising:
- coefficients of coancestry, each coefficient of coancestry indicates the probability that one feature [e.g. an allele], derived from the same common ancestor, is identical by descent in two individuals; and
- scores computed as a function of the coefficients of coancestry, in particular inbreeding coefficients, each inbreeding coefficient being a measure of inbreeding derived from a known genealogical relationship of the parents expressed in the form of coefficients of coancestry.

7. The computer-implemented method of any one of the previous claims, further comprising:
- computing each of the similarity scores in the incomplete set of similarity scores as a function of genetic, metabolic, transcription-related, protein-related and/or phenotypic markers of the two plant breeding units comprised in the plant breeding unit pair for which the similarity score is computed, the similarity scores being indicative of a degree of similarity of the markers of the two plant breeding units.

8. The computer-implemented method of any one of the previous claims, the similarity score being selected from a group comprising:
- a marker-based similarity score, in particular a genomic relationship score computed from DNA marker information;
- a marker co-occurrence score;
wherein the marker is selected from a group comprising:
- a genetic, metabolic, transcription-related, protein-related, phenotype-related marker and/or breeding value of a plant used as one of the plant breeding units; or
- an aggregate value derived from genetic, metabolic, transcription-related, protein-related, phenotypic markers and/or or breeding value of a group of plants used as one of the plant breeding units;

9. The computer-implemented method of any one of the previous claims, the plant breeding unit being groups of plants, each one of the groups of plants being selected from a group comprising:
- a group of plants having the same or a highly similar genotype that is different from the genotype of some or all other ones of the plant groups;
- a group of plants belonging to the same cultivar, the cultivar being different from the cultivar to which the plants of some or all of the other plant groups belong to.

10. The computer-implemented method of any one of the previous claims, further comprising:
- performing a cluster analysis on a base population of plant breeding units, thereby identifying a number n of clusters, each cluster comprising a sub-set of plant breeding units whose genetic, metabolic, transcription-related, protein-related phenotype-related and/or breeding-related markers are more similar to one another than to respective markers of plant breeding units of other ones of the clusters;
- for each of the number n of identified clusters:
∘ identifying pairs of plant breeding units comprised in this cluster;
∘ receiving pedigree scores for each of the identified pairs;
∘ receiving similarity scores of at least some of the identified pairs;
∘ aligning the pedigree scores and the similarity scores of identical plant breeding unit pairs selectively for the pairs in the cluster;
∘ performing an automated analysis of the aligned pedigree scores and similarity scores for determining associations of the similarity scores and of the pedigree scores in the cluster, thereby computing a cluster-specific predictive model, the cluster-specific predictive model being adapted to estimate a similarity score as a function of a pedigree score.

11. The computer-implemented method of claim 10, wherein the complete set of similarity scores (400, 518) is a set of preliminary similarity scores computed using the predictive model as a preliminary global predictive model, the method further comprising:
- applying the cluster-specific predictive models on pedigree scores of the sub-set of the plant breeding unit pairs of the one of the clusters from which the cluster-specific predictive model was derived for computing missing similarity scores for intra-cluster plant breeding unit pairs of the cluster;
- supplementing the received incomplete set of similarity scores with the similarity scores computed for the intra-cluster plant breeding unit pairs of the one or more clusters, thereby providing an intermediate incomplete set of similarity scores, the intermediate incomplete set of similarity scores being devoid of similarity scores of at least some of the inter-cluster plant breeding unit pairs;
- supplementing the intermediate incomplete set of similarity scores by using the preliminary similarity scores similarity scores as the missing similarity scores of the inter-cluster plant breeding unit pairs, thereby providing a refined complete set of similarity scores; and
- using the refined complete set of similarity scores for performing the computational prediction of the feature.

12. The computer-implemented method of claim 10, further comprising:
- applying the cluster-specific predictive models on pedigree scores of the sub-set of the plant breeding unit pairs of the one of the clusters from which the cluster-specific predictive model was derived for computing missing similarity scores for intra-cluster plant breeding unit pairs of the cluster;
- supplementing the received incomplete set of similarity scores with the similarity scores computed for the intra-cluster plant breeding unit pairs of the one or more clusters, thereby providing an intermediate incomplete set of similarity scores, the intermediate incomplete set of similarity scores being devoid of similarity scores of at least some inter-cluster plant breeding unit pairs;
- performing the method according to any one of the previous claims 1 - 9, thereby using the intermediate incomplete set of similarity scores as the received incomplete set of similarity scores, whereby the predictive model is computed by analyzing the aligned pedigree scores and the similarity scores of the intermediate incomplete set of similarity scores, whereby the computed predictive model is applied on the pedigree scores of inter-cluster plant breeding unit pairs for creating the complete set of similarity scores that is used for computationally predicting the feature.

13. The computer-implemented method of any one of the previous claims, wherein a base population of plant breeding units is used as the founding population of a pedigree tree from which the pedigree scores are derived, wherein the base population comprises at least two genetically distinct groups of plant breeding units.

14. The computer-implemented method of any one of the previous claims, wherein the predicted feature is selected from a group comprising:
- a breeding value of one or more of the plant breeding units;
- an identifier of one or more of the plant breeding units having the highest likelihood of comprising a favorable genomic, metabolic, or phenotypic marker;
- an identifier of one or more of the plant breeding units having the highest likelihood of comprising an undesired genomic, metabolic, or phenotypic marker;
- an identifier of at least one plant breeding unit pair comprising a favorable combination of genomic, metabolic, or phenotypic markers;
- an identifier of at least one plant breeding unit pair comprising an undesired combination of genomic, metabolic, or phenotypic markers;
- the likelihood of occurrence of a favorable or of an undesired genomic, metabolic, or phenotypic marker in an offspring of two of the plant breeding units.

15. A method for conducting a plant breeding project, the method comprising:
- providing a group of candidate plant breeding units, wherein a candidate plant breeding unit is an individual plant or a group of plants potentially to be used in the plant breeding project, wherein a known genealogical relationship of pairs of the candidate plant breeding units over two or more generations is available;
- performing the method according to anyone of the previous claims 1-14 for computationally predicting a feature of at least one of the candidate plant breeding units or of an offspring of at least one of the plant breeding units, wherein the candidate plant breeding units are used as the plant breeding units whose pedigree scores and incomplete set of similarity scores are received, wherein the feature is indicative of whether the at least one candidate breeding unit comprises a favorable genomic, metabolic, or phenotypic marker and/or a favorable breeding value;
- selecting one or more of the candidate breeding units in dependence on the at least one predicted feature; and
- selectively using the selected one or more candidate breeding units for generating offspring in the plant breeding project.

16. A computer-system (500) configured for predicting a feature of one or more plants, the computer system comprising:
- one or more processors;
- a volatile or non-volatile storage medium comprising:
∘ a set of pedigree scores (300, 512), the pedigree scores being indicative of known genealogical relationships of pairs of plant breeding units over two or more generations, the plant breeding unit pairs comprising pairs of plant breeding units within the same generation and comprising pairs of plant breeding units of different ones of the two or more generations, wherein a plant breeding unit is an individual plant or a group of plants;
∘ an incomplete set of similarity scores (200, 510), each similarity score being indicative of observed similarities between the two members of a respective one of the pairs of the plant breeding units, wherein the incomplete set of similarity scores is devoid of similarity scores of at least a sub-set of the plant breeding unit pairs;
∘ a software (502, 520) comprising computer-interpretable instructions which, when executed by the one or more processors, cause the processors to perform a method comprising:
▪ aligning (106) the pedigree scores and the similarity scores of identical plant breeding unit pairs;
▪ analyzing (108) the aligned pedigree scores and similarity scores for determining associations of the similarity scores and of the pedigree scores, thereby computing a predictive model, the predictive model being adapted to estimate a similarity score as a function of a pedigree score;
▪ applying (110) the predictive model on pedigree scores of the sub-set of the plant breeding unit pairs for computing missing similarity scores for each of the plant breeding unit pairs of the sub-set;
▪ creating (112) a complete set of similarity scores from the incomplete set of similarity scores and the computed missing similarity scores; and
▪ using (114) the complete set of similarity scores for computationally predicting a feature of at least one of the plant breeding units or of an offspring of at least one of the plant breeding units.
